(19) Europäisches Patentamt
European Patent Office
Office européen des brevets

(11) EP 0 942 905 B1

# (12) EUROPÄISCHE PATENTSCHRIFT

(45) Veröffentlichungstag und Bekanntmachung des Hinweises auf die Patenterteilung:
**20.02.2002 Patentblatt 2002/08**

(21) Anmeldenummer: **97951989.9**

(22) Anmeldetag: **27.11.1997**

(51) Int Cl.[7]: **C07D 239/54**, A01N 43/54

(86) Internationale Anmeldenummer:
**PCT/EP97/06618**

(87) Internationale Veröffentlichungsnummer:
**WO 98/25909 (18.06.1998 Gazette 1998/24)**

(54) **3-AMINO-1-CYANOPHENYL-URACILE**

3-AMINO-1-CYANOPHENYL-URACILS

3-AMINO-1-CYANOPHENYLE-URACILES

(84) Benannte Vertragsstaaten:
**BE CH DE DK ES FR GB IT LI NL**

(30) Priorität: **09.12.1996 DE 19651036**

(43) Veröffentlichungstag der Anmeldung:
**22.09.1999 Patentblatt 1999/38**

(73) Patentinhaber: **BAYER AG**
**51368 Leverkusen (DE)**

(72) Erfinder:
- **ANDREE, Roland**
  **D-40764 Langenfeld (DE)**
- **DREWES, Mark, Wilhelm**
  **D-40764 Langenfeld (DE)**
- **DOLLINGER, Markus**
  **D-51381 Leverkusen (DE)**

(56) Entgegenhaltungen:
**EP-A- 0 473 551** **DE-A- 19 604 229**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue 3-Amino-1-cyanophenyl-uracile, ein Verfahren zu ihrer Herstellung und ihre Verwendung als Herbizide.

Es ist bereits bekannt geworden, daß bestimmte 3-Alkyl-1-cyanophenyl-uracile herbizide Eigenschaften besitzen (vgl. EP-A 0 473 551 oder DE-A-196 04 229). So läßt sich zum Beispiel 2-Cyano-5-[3,6-dihydro-2,6-dioxo-3-methyl-4-trifluormethyl-1(2H)-pyrimidinyl]-4-fluor-benzoesäure-isopropylester zur Bekämpfung von Unkraut verwenden. Bei niedrigen Aufwandmengen ist die Wirksamkeit dieses Stoffes aber nicht immer befriedigend.

Es wurden nun neue 3-Amino-1-cyano-phenyl-uracile der Formel

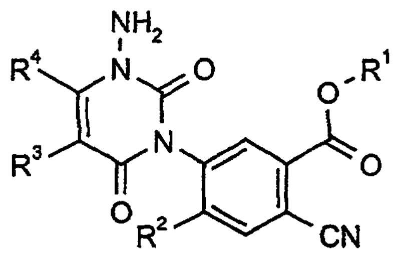

(I),

in welcher

- $R^1$ für Wasserstoff oder für gegebenenfalls durch Cyano, Carboxy, Halogen, $C_1$-$C_4$-Alkoxy oder $C_1$-$C_4$-Alkoxy-carbonyl substituiertes Alkyl mit 1 bis 6 Kohlenstoffatomen steht oder
  für jeweils gegebenenfalls durch Cyano, Carboxy oder $C_1$-$C_4$-Alkoxy-carbonyl substituiertes Alkenyl oder Alkinyl mit jeweils 2 bis 6 Kohlenstoffatomen steht, oder
  für jeweils gegebenenfalls einfach bis vierfach, gleichartig oder verschieden durch Cyano, Carboxy, Halogen, $C_1$-$C_4$-Alkyl oder $C_1$-$C_4$-Alkoxy-carbonyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil steht, oder
  für jeweils gegebenenfalls einfach bis dreifach, gleichartig oder verschieden durch Nitro, Cyano, Carboxy, Halogen, $C_1$-$C_4$-Alkyl, Halogen-$C_1$-$C_4$-alkyl, $C_1$-$C_4$-Alkoxy, Halogen-$C_1$-$C_4$-alkoxy und/oder $C_1$-$C_4$-Alkoxy-carbonyl substituiertes Aryl oder Arylalkyl mit 6 oder 10 Kohlenstoffatomen in der Arylgruppe und gegebenenfalls 1 bis 4 Kohlenstoffatomen im Alkylteil steht, oder
  für jeweils gegebenenfalls einfach bis dreifach, gleichartig oder verschieden durch Cyano, Carboxy, Halogen, $C_1$-$C_4$-Alkyl, Halogen-$C_1$-$C_4$-alkyl, $C_1$-$C_4$-Alkoxy und/oder Halogen-$C_1$-$C_4$-alkoxy substituiertes Heterocyclyl oder Heterocyclylalkyl aus der Reihe Oxetanyl, Furyl, Furylmethyl, Tetrahydrofuryl, Tetrahydrofurylmethyl, Thienyl, Oxazolyl, Isoxazolyl, Thiazolyl, Pyrazolyl, Oxadiazolyl, Pyridinyl, Pyridinylmethyl steht,

- $R^2$ für Wasserstoff, Cyano, Fluor, Chlor oder Brom steht,

- $R^3$ für Wasserstoff, Fluor, Chlor, Brom oder für gegebenenfalls einfach bis fünffach, gleichartig oder verschieden durch Fluor, Chlor und/oder Brom substituiertes Alkyl mit 1 bis 4 Kohlenstoffatomen steht, und

- $R^4$ für Alkyl mit 1 bis 4 Kohlenstoffatomen oder für Halogenalkyl mit 1 bis 4 Kohlenstoffatomen und 1 bis 5 Fluor-, Chlor- und/oder Bromatomen steht.

Weiterhin wurde gefunden, daß man 3-Amino-1-cyanophenyl-uracile der Formel (I) erhält, wenn man 1-Cyanophenyl-uracile der Formel

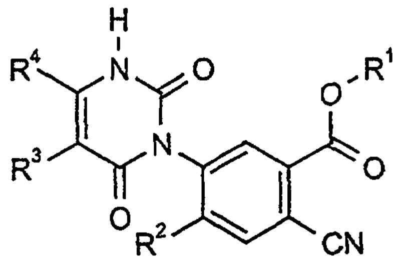

(II),

in welcher
$R^1$, $R^2$, $R^3$ und $R^4$ die oben angegebenen Bedeutungen haben,
mit einem elektrophilen Aminierungsmittel,
gegebenenfalls in Gegenwart eines Säurebindemittels und gegebenenfalls in Gegenwart eines Verdünnungsmittels umsetzt.

Schließlich wurde gefunden, daß die neuen 3-Amino-1-cyanophenyl-uracile der Formel (I) sehr gute herbizide Eigenschaften besitzen.

Überraschenderweise zeigen die erfindungsgemäßen 3-Amino-1-cyanophenyl-uracile der Formel (I) eine wesentlich bessere herbizide Wirksamkeit als der 2-Cyano-5-[3,6-dihydro-2,6-dioxo-3-methyl-4-trifluormethyl-1(2H)-pyrimidinyl]-4-fluor-benzoesäure-isopropylester, welches ein konstitutionell ähnlicher, vorbekannter Wirkstoff gleicher Wirkungsrichtung ist.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die erfindungsgemäßen 3-Amino-1-cyanophenyl-uracile sind durch die Formel (I) allgemein definiert. Bevorzugt sind Verbindungen der Formel (I), in denen

$R^1$ fur Wasserstoff oder fiir gegebenenfalls durch Cyano, Carboxy, Fluor, Chlor, Methoxy, Ethoxy, Propoxy, Methoxycarbonyl, Ethoxycarbonyl oder Propoxycarbonyl substituiertes Methyl, Ethyl, n- oder i-Propyl, n-, i-, s- oder t-Butyl steht, oder
für jeweils gegebenenfalls durch Cyano, Carboxy, Methoxycarbonyl oder Ethoxycarbonyl substituiertes Propenyl, Butenyl, Propinyl oder Butinyl steht, oder
für jeweils gegebenenfalls einfach bis vierfach, gleichartig oder verschieden durch Cyano, Carboxy, Fluor, Chlor, Methyl, Ethyl, Methoxycarbonyl oder Ethoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht, oder
für jeweils gegebenenfalls einfach bis dreifach, gleichartig oder verschieden durch Nitro, Cyano, Carboxy, Fluor, Chlor, Methyl, Ethyl, Trifluormethyl, Methoxy, Ethoxy, Difluormethoxy, Trifluormethoxy, Methoxycarbonyl und/oder Ethoxycarbonyl substituiertes Phenyl oder Benzyl steht, oder
fiir jeweils gegebenenfalls einfach bis dreifach, gleichartig oder verschieden durch Cyano, Carboxy, Fluor, Chlor, Methyl, Ethyl, Trifluormethyl, Methoxy, Ethoxy, Difluormethoxy und/oder Trifluormethoxy substituiertes Heterocyclyl oder Heterocyclylalkyl aus der Reihe Oxetanyl, Furyl, Furylmethyl, Tetrahydrofuryl, Tetrahydrofurylmethyl, Thienyl, Oxazolyl, Isoxazolyl, Thiazolyl, Pyrazolyl, Oxadiazolyl, Pyridinyl, Pyridinylmethyl steht,

$R^2$ für Wasserstoff, Fluor oder Chlor steht,

$R^3$ fiir Wasserstoff, Fluor, Chlor, Methyl, Ethyl oder fiir Halogenalkyl mit 1 oder 2 Kohlenstoffatomen und 1 bis 3 Fluor- und/oder Chloratomen steht und

$R^4$ für Methyl, Ethyl oder für Halogenalkyl mit 1 oder 2 Kohlenstoffatomen und 1 bis 5 Fluor- und/oder Chloratomen steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen auf-

geführt.

Gruppe 1

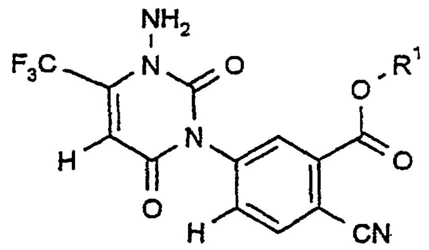

(Ia)

$R^1$ hat hierbei die folgenden Bedeutungen:
Wasserstoff, Methyl, Ethyl, n- oder i-Propyl, n-, i-, s- oder t-Butyl, n-, i-, s- oder t-Pentyl, Fluorethyl, Chlorethyl, Chlorfluorethyl, Difluorethyl, Dichlorethyl, Trifluorethyl, Trichlorethyl, Chlordifluorethyl, Fluorpropyl, Chlorpropyl, Difluorpropyl, Dichlorpropyl, Trifluorpropyl, Trichlorpropyl, Cyanoethyl, Cyanopropyl, Cyanobutyl, Carboxymethyl, Carboxyethyl, Carboxypropyl, Carboxybutyl, Methoxymethyl, Ethoxymethyl, Propoxymethyl, Methoxyethyl, Ethoxyethyl, Propoxyethyl, Methoxypropyl, Ethoxypropyl, Propoxypropyl, Methoxycarbonylmethyl, Ethoxycarbonylmethyl, Propoxycarbonylmethyl, Methoxycarbonylethyl, Ethoxycarbonylethyl, Propoxycarbonylethyl, Methoxycarbonylpropyl, Ethoxycarbonylpropyl, Propoxycarbonylpropyl, 1-Propen-3-yl (Allyl), 3-Methyl-1-propen-3-yl, 2-Buten-4-yl (Crotonyl), 1-Propin-3-yl (Propargyl), 3-Methyl-1-propin-3-yl, 2-Butin-4-yl, Cyclopropyl, Cyanocyclopropyl, Carboxycyclopropyl, Difluorcyclopropyl, Dichlorcyclopropyl, Methylcyclopropyl, Methoxycarbonylcyclopropyl, Ethoxycarbonylcyclopropyl, Cyclobutyl, Cyanocyclobutyl, Carboxycyclobutyl, Difluorcyclopropyl, Trifluorcyclobutyl, Tetrafluorcyclobutyl, Chlortrifluorcyclobutyl, Methylcyclobutyl, Cyclopentyl, Cyanocyclopentyl, Carboxycyclopentyl, Fluorcyclopentyl, Chlorcyclopentyl, Difluorcyclopentyl, Dichlorcyclopentyl, Methylcyclopentyl, Methoxycarbonylcyclopentyl, Ethoxycarbonylcyclopentyl, Cyclohexyl, Cyanocyclohexyl, Carboxycyclohexyl, Fluorcyclohexyl, Chlorcyclohexyl, Difluorcyclohexyl, Dichlorcyclohexyl, Methylcyclohexyl, Trifluormethylcyclohexyl, Methoxycarbonylcyclohexyl, Ethoxycarbonylcyclohexyl, Cyclopropylmethyl, Difluorcyclopropylmethyl, Dichlorcyclopropylmethyl, Cyclobutylmethyl, Cyclopentylmethyl, Cyclohexylmethyl, Cyanocyclohexylmethyl, Carboxycyclohexylmethyl, Fluorcyclohexylmethyl, Chlorcyclohexylmethyl, Methylcyclohexylmethyl, Trifluormethylcyclohexylmethyl, Phenyl, Cyanophenyl, Carboxyphenyl, Nitrophenyl, Fluorphenyl, Chlorphenyl, Bromphenyl, Methylphenyl, Trifluormethylphenyl, Methoxyphenyl, Difluormethoxyphenyl, Trifluormethoxyphenyl, Methoxycarbonylphenyl, Ethoxycarbonylphenyl, Benzyl, Cyanobenzyl, Carboxybenzyl, Fluorbenzyl, Chlorbenzyl, Methylbenzyl, Trifluormethylbenzyl, Methoxybenzyl, Difluormethoxybenzyl, Trifluormethoxybenzyl, Methoxycarbonylbenzyl, Ethoxycarbonylbenzyl, Furyl, Tetrahydrofuryl, Thienyl, Tetrahydrothienyl, Oxetanyl, Oxazolyl, Isoxazolyl.

Gruppe 2

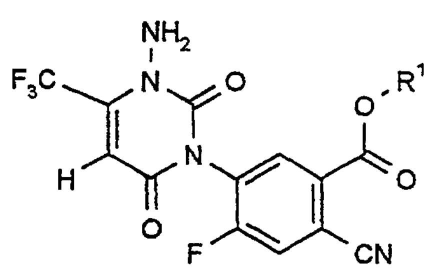

(Ib)

$R^1$ hat hierbei die oben in Gruppe 1 aufgeführten Bedeutungen.

Gruppe 3

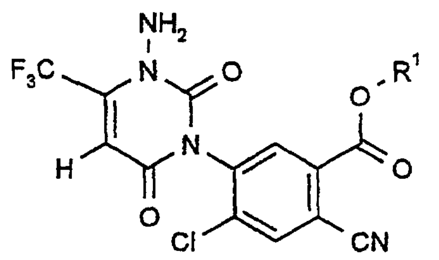

(Ic)

$R^1$ hat hierbei die oben in Gruppe 1 aufgeführten Bedeutungen.

Gruppe 4

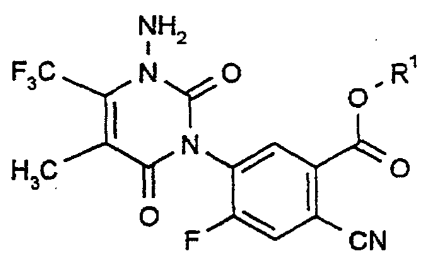

(Id)

$R^1$ hat hierbei die oben in Gruppe 1 aufgeführten Bedeutungen.

Gruppe 5

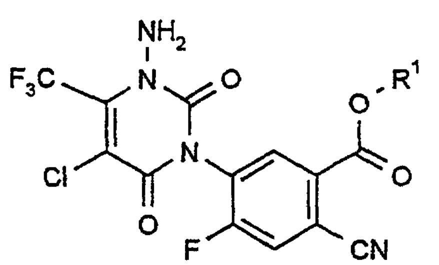

(Ie)

$R^1$ hat hierbei die oben in Gruppe 1 aufgeführten Bedeutungen.

Gruppe 6

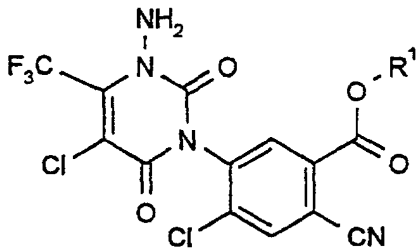

(If)

$R^1$ hat hierbei die oben in Gruppe 1 aufgeführten Bedeutungen.

Gruppe 7

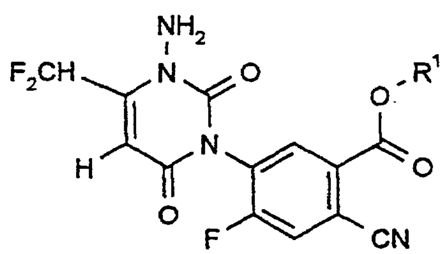

(Ig)

$R^1$ hat hierbei die oben in Gruppe 1 aufgeführten Bedeutungen.

Gruppe 8

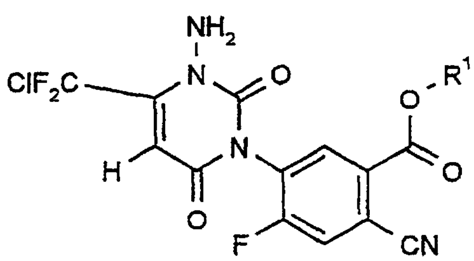

(Ih)

$R^1$ hat hierbei die oben in Gruppe 1 aufgeführten Bedeutungen.

Gruppe 9

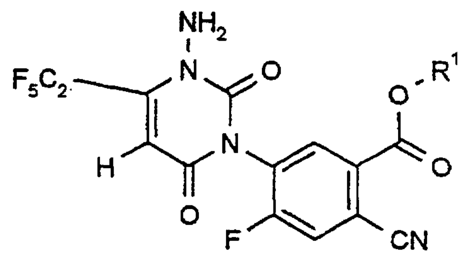

(Ik)

$R^1$ hat hierbei die oben in Gruppe 1 aufgeführten Bedeutungen.

Verwendet man 1-(4-Cyano-3-methoxycarbonyl-phenyl)-3,6-dihydro-2,6-dioxo-4-trifluormethyl-1(2H)-pyrimidin als Ausgangsstoff und 1-Aminooxy-2,4-dinitro-benzol als elektrophiles Aminierungsmittel, so kann der Reaktionsablauf beim erfindungsgemäßen Verfahren durch das folgende Formelschema skizziert werden:

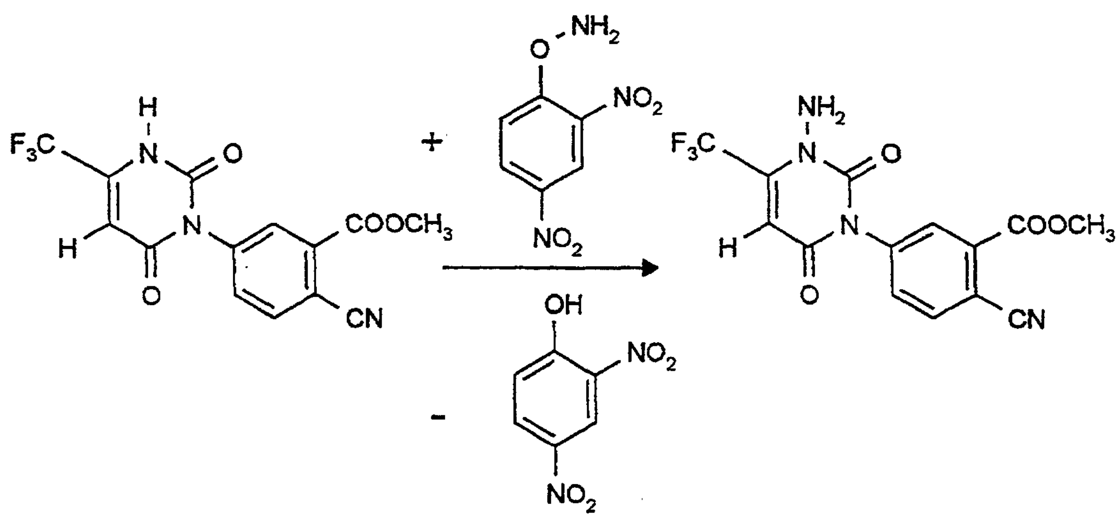

Die bei der Durchführung des erfindungsgemäßen Verfahrens als Ausgangsstoffe benötigten 1-Cyanophenyl-uracile sind durch die Formel (II) allgemein definiert. In dieser Formel haben $R^1$, $R^2$, $R^3$ und $R^4$ vorzugsweise bzw. besonders bevorzugt diejenigen Bedeutungen, die bereits im Zusammenhang mit der Beschreibung der erfindungsgemäßen Stoffe der Formel (I) vorzugsweise bzw. als besonders bevorzugt für diese Reste genannt wurden.

Die 1-Cyanophenyl-uracile der Formel (II) sind bekannt oder lassen sich nach prinzipiell bekannten Verfahren herstellen (vgl. EP-A 0 473 551).

Als elektrophile Aminierungsmittel kommen bei der Durchführung des erfindungsgemäßen Verfahrens alle üblichen Substanzen in Frage, die zur elektrophilen Einführung einer Aminogruppe geeignet sind. Als Beispiele hierfür seien 1-Aminooxy-2,4-dinitrobenzol (= 2,4-Dinitro-phenyl-hydroxylamin) und Hydroxylamin-O-sulfonsäure genannt.

Als Säurebindemittel kommen bei der Durchführung des erfindungsgemäßen Verfahrens alle üblichen anorganischen und organischen Basen in Betracht. Vorzugsweise verwendbar sind Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo [5,4,0]-undec-7-en (DBU).

Als Verdünnungsmittel kommen bei der Durchführung des erfindungsgemäßen Verfahrens alle üblichen inerten, organischen Solventien in Frage. Vorzugsweise verwendbar sind aliphatische, alicyclische und aromatische, gegebenenfalls halogenierte Kohlenwasserstoffe, wie Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, ferner Dialkylether, wie Diethylether, Diisopropylether, Methyl-t-butylether, Ethyl-t-butylether, Methyl-t-pentylether (MTBE), Ethyl-t-pentylether, Tetrahydrofuran (THF), 1,4-Dioxan, Ethylenglycol-dimethylether oder -diethylether, Diethylenglycol-dimethylether oder -diethylether; außerdem Dialkylketone, wie Aceton, Butanon (Methylethylketon), Methyl-i-propylketon oder Methyl-i-butylketon, weiterhin Nitrile, wie Acetonitril, Propionitril, Butyronitril oder Benzonitril; ferner Amide, wie N,N-Dimethyl-formamid (DMF), N,N-Dimethyl-acetamid, N-Methyl-formanilid, N-Methyl-pyrrolidon oder Hexamethyl-phosphorsäuretriamid; außerdem Ester, wie Essigsäure-methylester, -ethylester, -n- oder -i-propylester, -n-, -i- oder -s-butylester; weiterhin Sulfoxide, wie Dimethylsulfoxid; und auch Alkanole, wie Methanol, Ethanol, n- oder i-Propanol, n-, i-, s- oder t-Butanol, Ethylenglycol-monomethylether oder -monoethylether, Diethylenglycol-monomethylether oder -monoethylether; sowie deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen -20°C und +100°C, vorzugsweise zwischen 0°C und 80°C, insbesondere zwischen 20°C und 60°C.

Das erfindungsgemäße Verfahren wird im allgemeinen unter Atmosphärendruck durchgeführt. Es ist jedoch auch möglich, unter erhöhtem oder vermindertem Druck, - im allgemeinen zwischen 0,1 bar und 10 bar -, zu arbeiten.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Säurebindemittels durchgeführt, und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen und überjährigen Kulturen eingesetzt werden.

Die erfindungsgemäßen Verbindungen der Formel (I) eignen sich sehr gut zur Bekämpfung von monokotylen und dikotylen Unkräutern sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate,

Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Dabei kann in manchen Fällen auch Synergismus auftreten.

Für die Mischungen kommen beispielsweise folgende Herbizide in Betracht:

Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofumesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(-ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-Verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

**Herstellungsbeispiele:**

**Beispiel 1**

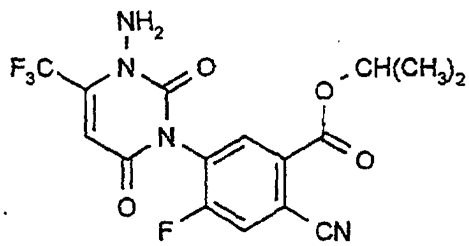

Eine Mischung aus 1,3 g (3,38 mMol) 1-(4-Cyano-2-fluor-5-i-propoxycarbonylphenyl)-3,6-dihydro-2,6-dioxo-4-trifluormethyl-1(2H)-pyrimidin, 0,76 g (3,38 mMol) 1-Aminooxy-2,4-dinitro-benzol, 0,33 g Natriumhydrogencarbonat und 10 ml N,N-Dimethyl-formamid wird 12 Tage bei Raumtemperatur (ca. 20°C) gerührt, wobei jeden zweiten Tag 1,2 g 1-Aminooxy-2,4-dinitro-benzol und 1,0 g Natriumhydrogencarbonat hinzugefügt werden. Die Mischung wird dann auf 0,1%ige wässrige Natronlauge gegeben und mit Diethylether extrahiert. Die organische Phase wird zunächst mit 0,1%iger Natronlauge und dann mit 1N-Salzsäure gewaschen, mit Natriumsulfat getrocknet und filtriert. Das Filtrat wird im Wasserstrahlvakuum eingeengt, der Rückstand mit 3 ml Diethylether digeriert und das kristallin angefallene Produkt durch Absaugen isoliert.

Man erhält 0,45 g (33% der Theorie) 3-Amino-1-(4-cyano-2-fluor-5-i-propoxycarbonyl-phenyl)-3,6-dihydro-2,6-dioxo-4-trifluormethyl-1(2H)-pyrimidin vom Schmelzpunkt 166°C.

**Herstellung der Ausgangssubstanz:**

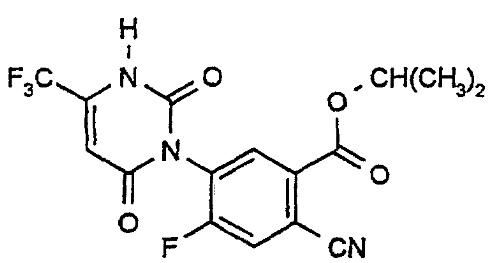

(II-1)

3,0 g (13,5 mMol) 5-Amino-2-cyano-4-fluor-benzoesäure-i-propylester werden in 50 ml Aceton gelöst und dann mit 3,2 g (16 mMol) Chlorameisensäure-trichlormethylester ("Diphosgen") tropfenweise versetzt. Die Mischung wird eine Stunde bei Raumtemperatur gerührt und anschließend im Wasserstrahlvakuum eingeengt. Der Rückstand wird in Toluol gelöst und dann bei -30°C zu einer Mischung aus 3,3 g (13,5 mMol) 3-Amino-4,4,4-trifluor-crotonsäure-ethylester, 0,52 g Natriumhydrid, 20 ml Toluol und 30 ml N,N-Dimethyl-formamid tropfenweise gegeben. Die Reaktionsmischung wird 20 Minuten bei -30°C gerührt, dann auf Wasser gegeben, mit Diethylether geschüttelt, mit IN-Salzsäure angesäuert und mit Essigsäureethylester geschüttelt. Die organische Phase wird mit Wasser gewaschen, mit Natriumsulfat getrocknet und filtriert. Das Filtrat wird im Wasserstrahlvakuum eingeengt, der Rückstand mit Diethylether digeriert und das kristallin angefallende Produkt durch Absaugen isoliert.

Man erhält 1,6 g (32% der Theorie) 1-(4-Cyano-2-fluor-5-i-propoxycarbonyl-phenyl)-3,6-dihydro-2,6-dioxo-4-trifluormethyl-1(2H)-pyrimidin vom Schmelzpunkt 120°C.

Nach den zuvor angegebenen Methoden lassen sich auch die in der folgenden Tabelle 1 aufgeführten Verbindungen der Formel (I) herstellen.

**Tabelle 1**

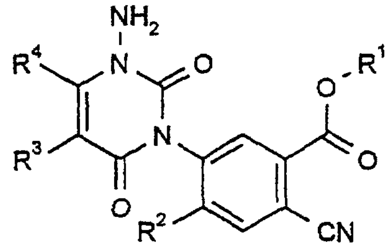

(I)

| Bsp.-Nr. | $R^1$ | $R^2$ | $R^3$ | $R^4$ | Physikal. Daten |
|---|---|---|---|---|---|
| 2 | $CH_3$ | F | H | $CF_3$ | |
| 3 | $C_2H_5$ | F | H | $CF_3$ | |
| 4 | 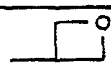 | F | H | $CF_3$ | |
| 5 | $C_3H_7$-n | F | H | $CF_3$ | |
| 6 | 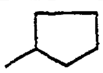 | F | H | $CF_3$ | |
| 7 | 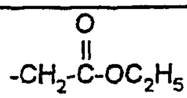 | F | H | $CF_3$ | |
| 8 | 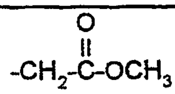 | F | H | $CF_3$ | |
| 9 | 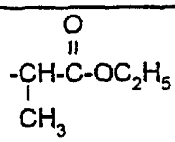 | F | H | $CF_3$ | |
| 10 | 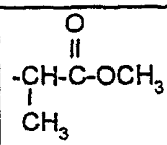 | F | H | $CF_3$ | |
| 11 | 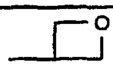 | Cl | H | $CF_3$ | |
| 12 | $C_3H_7$-i | H | H | $CF_3$ | |
| 13 | $-OCH_2CH_2OCH_3$ | F | H | $CF_3$ | |
| 14 | $-CH_2-CH=CH_2$ | F | H | $CF_3$ | |
| 15 | 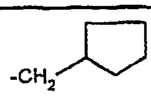 | F | H | $CF_3$ | |

**Tabelle 1** (Fortsetzung)

| Bsp.-Nr. | $R^1$ | $R^2$ | $R^3$ | $R^4$ | Physikal. Daten |
|---|---|---|---|---|---|
| 16 | 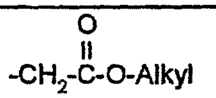 | F | H | $CF_3$ | |
| 17 | 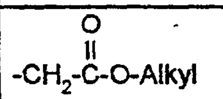 | H | H | $CF_3$ | |
| 18 | 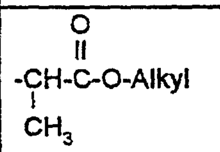 | F | H | $CF_3$ | |
| 19 | 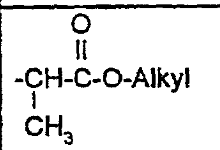 | H | H | $CF_3$ | |

**Verwendungsbeispiele:**

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Samen der Testpflanzen werden in normalen Boden ausgesät. Nach ca. 24 Stunden wird der Boden mit der Wirkstoffzubereitung so gespritzt, daß die jeweils gemischten Wirkstoffmengen pro Flächeneinheit ausgebracht werden. Die Konzentration der Spritzbrühe wird so gewählt, daß in 1 0001 Wasser/ha die jeweils gewünschten Wirkstoffmengen ausgebracht werden.
Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)

100% = totale Vernichtung

In diesem Test zeigt die Verbindung gemäß Herstellungsbeispiel 1 bei einer Aufwandmenge von 30 g/ha eine Wirkung von mehr als 90 % gegen mehrere Unkräuter.

**Tabelle A:** Pre-emergence-Test/Gewächshaus

| Verbindung gemäß Herstellungsbeispiel | Aufwand-menge (g ai./ha) | Echino-chloa | Sorghum | Abutilon | Datura | Matri-caria |
|---|---|---|---|---|---|---|
| 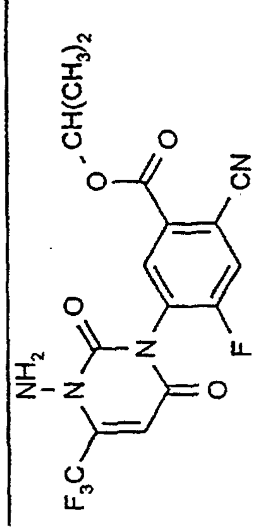 (1) | 30 | 100 | 95 | 100 | 100 | 100 |

**Beispiel B**

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigt die Verbindung gemäß Herstellungsbeispiel 1 bei einer Aufwandmenge von 30 g/ha gegen mehrere Unkräuter eine Wirkung von 100 %.

**Tabelle B:** Post-emergence-Test/Gewächshaus

| Verbindung gemäß Herstellungsbeispiel | Aufwand-menge (g ai./ha) | Alope-curus | Bromus | Setaria | Abutilon | Datura | Matri-caria |
|---|---|---|---|---|---|---|---|
| 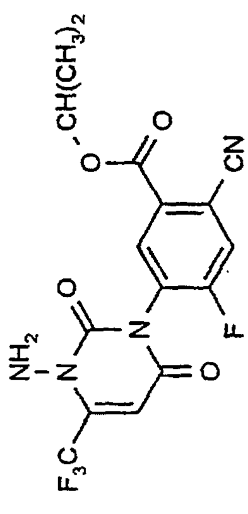 (1) | 30 | 100 | 100 | 100 | 100 | 100 | 100 |

## Patentansprüche

**1.** 3-Amino-1-cyanophenyl-uracile der Formel

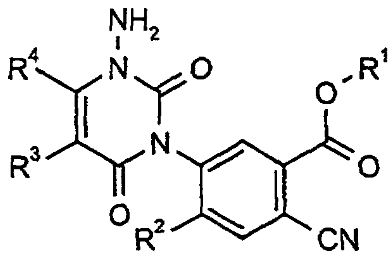

(I),

in welcher

$R^1$ für Wasserstoff oder für gegebenenfalls durch Cyano, Carboxy, Halogen, $C_1$-$C_4$-Alkoxy oder $C_1$-$C_4$-Alkoxy-carbonyl substituiertes Alkyl mit 1 bis 6 Kohlenstoffatomen steht oder
für jeweils gegebenenfalls durch Cyano, Carboxy oder $C_1$-$C_4$-Alkoxy-carbonyl substituiertes Alkenyl oder Alkinyl mit jeweils 2 bis 6 Kohlenstoffatomen steht, oder
für jeweils gegebenenfalls einfach bis vierfach, gleichartig oder verschieden durch Cyano, Carboxy, Halogen, $C_1$-$C_4$-Alkyl oder $C_1$-$C_4$-Alkoxy-carbonyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil steht, oder
fiir jeweils gegebenenfalls einfach bis dreifach, gleichartig oder verschieden durch Nitro, Cyano, Carboxy, Halogen, $C_1$-$C_4$-Alkyl, Halogen-$C_1$-$C_4$-alkyl, $C_1$-$C_4$-Alkoxy, Halogen-$C_1$-$C_4$-alkoxy und/oder $C_1$-$C_4$-Alkoxy-carbonyl substituiertes Aryl oder Arylalkyl mit 6 oder 10 Kohlenstoffatomen in der Arylgruppe und gegebenenfalls 1 bis 4 Kohlenstoffatomen im Alkylteil steht, oder
für jeweils gegebenenfalls einfach bis dreifach, gleichartig oder verschieden durch Cyano, Carboxy, Halogen, $C_1$-$C_4$-Alkyl, Halogen-$C_1$-$C_4$-alkyl, $C_1$-$C_4$-Alkoxy und/oder Halogen-$C_1$-$C_4$-alkoxy substituiertes Heterocyclyl oder Heterocyclylalkyl aus der Reihe Oxetanyl, Furyl, Furylmethyl, Tetrahydrofuryl, Tetrahydrofurylmethyl, Thienyl, Oxazolyl, Isoxazolyl, Thiazolyl, Pyrazolyl, Oxadiazolyl, Pyridinyl, Pyridinylmethyl steht,

$R^2$ fiir Wasserstoff, Cyano, Fluor, Chlor oder Brom steht,

$R^3$ für Wasserstoff, Fluor, Chlor, Brom oder für gegebenenfalls einfach bis fünffach, gleichartig oder verschieden durch Fluor, Chlor und/oder Brom substituiertes Alkyl mit 1 bis 4 Kohlenstoffatomen steht, und

$R^4$ fiir Alkyl mit 1 bis 4 Kohlenstoffatomen oder für Halogenalkyl mit 1 bis 4 Kohlenstoffatomen und 1 bis 5 Fluor-, Chlor- und/oder Bromatomen steht.

2. Verfahren zur Herstellung von 3-Amino-1-cyanophenyl-uracilen der Formel (I) gemäß Anspruch 1, **dadurch gekennzeichnet, daß** man 3-Amino-1-cyanophenyl-uracile der Formel (I) erhält, wenn man 1-Cyanophenyl-uracile der Formel

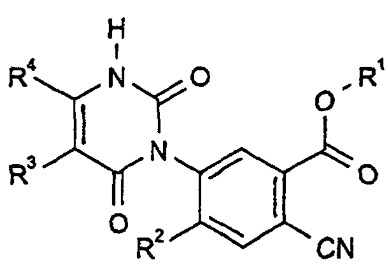

(II),

in welcher
$R^1$, $R^2$, $R^3$ und $R^4$ die oben angegebenen Bedeutungen haben,
mit einem elektrophilen Aminierungsmittel,
gegebenenfalls in Gegenwart eines Säurebindemittels und gegebenenfalls in Gegenwart eines Verdünnungsmittels umsetzt.

**3.** Herbizide Mittel, **gekennzeichnet durch** einen Gehalt an mindestens einem 3-Amino-1-cyanophenyl-uracil der Formel (I) gemäß Anspruch 1.

**4.** Verwendung von 3-Amino-1-cyanophenyl-uracilen der Formel (I) gemäß Anspruch 1 zur Bekämpfung von Unkräutern.

**5.** Verfahren zur Bekämpfung von Unkräutern, **dadurch gekennzeichnet, daß** man 3-Amino-1-cyanophenyl-uracile der Formel (I) gemäß Anspruch 1 auf die Unkräuter und/oder deren Lebensraum ausbringt.

**6.** Verfahren zur Herstellung von herbiziden Mitteln, **dadurch gekennzeichnet, daß** man 3-Amino-1-cyanophenyl-uracile der Formel (I) gemäß Anspruch 1 mit Streckmitteln und/oder oberflächenaktiven Stoffen vermischt.

**Claims**

**1.** 3-Amino-1-cyanophenyl-uracils of the formula

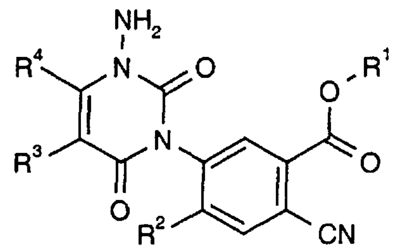

(I),

in which

$R^1$ represents hydrogen or represents optionally cyano-, carboxyl-, halogen-, $C_1$-$C_4$-alkoxy- or $C_1$-$C_4$-alkoxy-carbonyl-substituted alkyl having 1 to 6 carbon atoms or
represents in each case optionally cyano-, carboxyl- or $C_1$-$C_4$-alkoxy-carbonyl-substituted alkenyl or alkinyl having in each case 2 to 6 carbon atoms, or
represents cycloalkyl or cycloalkylalkyl having in each case 3 to 6 carbon atoms in the cycloalkyl group and optionally 1 to 4 carbon atoms in the alkyl moiety and being in each case optionally mono- to tetrasubstituted by identical or different substituents selected from the group consisting of cyano, carboxyl, halogen, $C_1$-$C_4$-alkyl and $C_1$-$C_4$-alkoxy-carbonyl, or
represents aryl or arylalkyl having 6 or 10 carbon atoms in the aryl group and optionally 1 to 4 carbon atoms in the alkyl moiety and being in each case optionally mono- to trisubstituted by identical or different substituents selected from the group consisting of nitro, cyano, carboxyl, halogen, $C_1$-$C_4$-alkyl, halogeno-$C_1$-$C_4$-alkyl, $C_1$-$C_4$-alkoxy, halogeno-$C_1$-$C_4$-alkoxy and/or $C_1$-$C_4$-alkoxy-carbonyl, or
represents heterocyclyl or heterocyclylalkyl from the series oxetanyl, furyl, furylmethyl, tetrahydrofuryl, tetrahydrofurylmethyl, thienyl, oxazolyl, isoxazolyl, thiazolyl, pyrazolyl, oxadiazolyl, pyridinyl, pyridinylmethyl, each of which is optionally mono- to trisubstituted by identical or different substituents selected from the group consisting of cyano, carboxyl, halogen, $C_1$-$C_4$-alkyl, halogeno-$C_1$-$C_4$-alkyl, $C_1$-$C_4$-alkoxy and/or halogeno-$C_1$-$C_4$-alkoxy,

$R^2$ represents hydrogen, cyano, fluorine, chlorine or bromine,

$R^3$ represents hydrogen, fluorine, chlorine, bromine or represents alkyl having 1 to 4 carbon atoms which is optionally mono- to pentasubstituted by identical or different substituents selected from the group consisting of fluorine, chlorine and bromine, and

$R^4$ represents alkyl having 1 to 4 carbon atoms or represents halogenoalkyl having 1 to 4 carbon atoms and 1 to 5 fluorine, chlorine and/or bromine atoms.

**2.** Process for preparing 3-amino-1-cyanophenyl-uracils of the formula (I) according to Claim 1, **characterized in**

**that** 3-amino-1-cyanophenyl-uracils of the formula (I) are obtained when 1-cyanophenyl-uracils of the formula

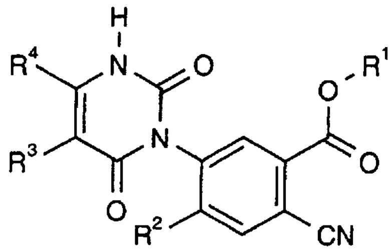

(II),

in which
$R^1$, $R^2$, $R^3$ and $R^4$ are each as defined above,
are reacted with an electrophilic aminating agent,
if appropriate in the presence of an acid binder and if appropriate in the presence of a diluent.

**3.** Herbicidal composition, **characterized in that** it contains at least one 3-amino-1-cyanophenyl-uracil of the formula (I) according to Claim 1.

**4.** The use of 3-amino-1-cyanophenyl-uracils of the formula (I) according to Claim 1 for controlling weeds.

**5.** Method for controlling weeds, **characterized in that** 3-amino-1-cyanophenyl-uracils of the formula (I) according to Claim 1 are applied to the weeds and/or their habitat.

**6.** Process for preparing herbicidal compositions, **characterized in that** 3-amino-1-cyanophenyl-uracils of the formula (I) according to Claim 1 are mixed with extenders and/or surfactants.

## Revendications

**1.** 3-amino-1-cyanophényl-uraciles de formule

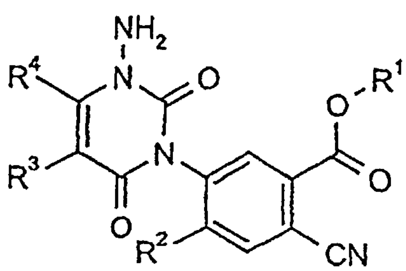

(I),

dans laquelle

$R^1$ représente l'hydrogène ou un groupe alkyle de 1 à 6 atomes de carbone portant éventuellement un substituant cyano, carboxy, halogéno, alkoxy en $C_1$ à $C_4$ ou (alkoxy en $C_1$ à $C_4$)-carbonyle, ou bien
un groupe alcényle ou un groupe alcynyle ayant chacun 2 à 6 atomes de carbone et portant chacun, le cas échéant, un substituant cyano, carboxy ou (alkyle en $C_1$ à $C_4$)-carbonyle, ou bien
un groupe cycloalkyle ou un groupe cycloalkylalkyle ayant chacun 3 à 6 atomes de carbone dans le groupe cycloalkyle et, le cas échéant, 1 à 4 atomes de carbone dans la partie alkyle et portant chacun, le cas échéant, un à quatre substituants, identiques ou différents, cyano, carboxy, halogéno, alkyle en $C_1$ à $C_4$ ou (alkoxy en $C_1$ à $C_4$)-carbonyle, ou bien
un groupe aryle ou un groupe arylalkyle ayant 6 ou 10 atomes de carbone dans le groupe aryle et, le cas échéant, 1 à 4 atomes de carbone dans la partie alkyle et portant chacun, le cas échéant, un à trois substituants, identiques ou différents, nitro, cyano, carboxy, halogéno, alkyle en $C_1$ à $C_4$, halogénalkyle en $C_1$ à $C_4$, alkoxy en $C_1$ à $C_4$, halogénalkoxy en $C_1$ à $C_4$ et/ou (alkoxy en $C_1$ à $C_4$)-carbonyle, ou bien un groupe hétérocyclyle ou un groupe hétérocyclylalkyle de la série oxétannyle, furyle, furylméthyle, tétrahydrofuryle, tétrahydrofurylméthyle, thiényle, oxazolyle, isoxazolyle, thiazolyle, pyrazolyle, oxadiazolyle, pyridinyle, pyridinylméthyle, portant chacun, le cas échéant, un à trois substituants, identiques ou différents, cyano, carboxy, halogéno, alkyle en $C_1$ à $C_4$, halogénalkyle en $C_1$ à $C_4$, alkoxy en $C_1$ à $C_4$ et/ou halogénalkoxy en $C_1$ à $C_4$,

$R^2$ représente l'hydrogène, un groupe cyano, le fluor, le chlore ou le brome,

$R^3$ représente l'hydrogène, le fluor, le chlore, le brome ou un groupe alkyle de 1 à 4 atomes de carbone portant éventuellement un à cinq substituants, identiques ou différents, fluoro, chloro et/ou bromo, et

$R^4$ représente un groupe alkyle ayant 1 à 4 atomes de carbone ou un groupe halogénalkyle ayant 1 à 4 atomes de carbone et 1 à 5 atomes de fluor, de chlore et/ou de brome.

2. Procédé de production de 3-amino-1-cyanophényl-uraciles de formule (I) suivant la revendication 1, **caractérisé en ce qu'**on obtient des 3-amine-1-cyanophényl-uraciles de formule (I) en faisant réagir des 1-cyanophényl-uraciles de formule

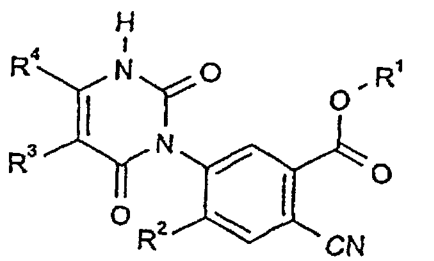

(II),

dans laquelle
$R^1$, $R^2$, $R^3$ et $R^4$ ont les définitions indiquées ci-dessus,
avec un agent d'amination électrophile, éventuellement en présence d'un accepteur d'acide et en présence éventuelle d'un diluant.

3. Compositions herbicides, **caractérisées par** une teneur en au moins un 3-amino-1-cyanophényl-uracile de formule (I) suivant la revendication 1.

4. Utilisation de 3-amino-1-cyanophényl-uraciles de formule (I) suivant la revendication 1 pour combattre des mauvaises herbes.

5. Procédé pour combattre des mauvaises herbes, **caractérisé en ce qu'**on applique sur les mauvaises herbes et/ou sur leur milieu des 3-amino-1-cyanophényl-uraciles de formule (I) suivant la revendication 1.

6. Procédé de préparation de compositions herbicides, **caractérisé en ce qu'**on mélange des 3-amino-1-cyanophényl-uraciles de formule (I) suivant la revendication 1 avec des diluants et/ou des agents tensio-actifs.